# EUROPEAN PATENT APPLICATION

(11) **EP 4 410 261 A1**
(43) Date of publication of application: **07.08.2024**
(21) Application number: 22875660.7
(22) Date of filing: 24.08.2022
(51) Int. Cl.: A61K 6/70, A61K 6/836, A61K 6/887, A61K 6/889

(54) **DENTAL COMPOSITION**

(30) Priority: 30.09.2021 JP 2021162412
(71) Applicant: GC Corporation, Sunto-gun, Shizuoka 410-1307 (JP)
(72) Inventor: SHIDA, Enzo, Tokyo 174-8585 (JP); TANAKA, Koji, Tokyo 174-8585 (JP)
(74) Representative: Müller-Boré & Partner Patentanwälte PartG mbB
(86) International application number: PCT/JP2022/031830
(87) International publication number: WO 2023/053788

(57) **Abstract**

A dental composition includes an acidic component; and at least one lanthanoid fluoride powder selected from the group consisting of fluoride powders of lanthanoids having an atomic number of 66 or less.

## Description

### Technical Field

The disclosures herein generally relate to a dental composition.

### Background Art

When dental materials consisting of a dental composition are used for an oral prosthesis, X-ray contrast (impermeability) is required for the dental composition so that the position of the material can be checked. As materials exhibiting X-ray contrast, ytterbium trifluoride (see, for example, Patent Document 1) and barium glass (see, for example, Patent Document 2) are known.

### Citation List

### Patent Document

Patent Document 1: Japanese Examined Patent Publication No. H7-76164
Patent Document 2: Japanese Unexamined Patent Application Publication No. 2000-102548

### Summary of Invention

### Technical Problem

However, some dental compositions contain an acidic component, and when the acidic component and ytterbium trifluoride or barium glass are contained together in an agent, a reaction occurs during storage, resulting in storage stability problems that prevent the agent from being used properly at a time of use.

An object of the present invention is to provide a dental composition that achieves both X-ray impermeability and storage stability.

### Solution to Problem

According to one aspect of the present disclosure, a dental composition includes: a dental composition including: an acidic component; and at least one lanthanoid fluoride powder selected from the group consisting of fluoride powders of lanthanoids having an atomic number of 66 or less.

### Advantageous Effects of Invention

According to one aspect of the present disclosure, a dental composition that achieves both X-ray impermeability and storage stability, can be provided.

### Description of Embodiments

In the following, embodiments of the present invention will be described in detail.

A dental composition according to the present embodiment includes an acidic component and lanthanoid fluoride powder.

As used herein, a dental composition refers to a composition used for dental materials.

The acidic component contained in the dental composition of the present embodiment is a component that has an acid group and becomes acidic (less than pH7.0) when dissolved in water. The acidic component is not particularly limited, but may be an inorganic acid or an organic acid.

Examples of the inorganic acid include a phosphoric acid, a sulfuric acid, a hydrochloric acid and a nitric acid. Among these, the phosphoric acid is preferable from the viewpoint of safety for the human body.

Examples of the organic acids include monocarboxylic acids such as formic acid, acetic acid, benzoic acid, and derivatives thereof; dicarboxylic acids such as oxalic acid, malonic acid, succinic acid, tartaric acid, adipic acid, pimelic acid, azelaic acid, sebacic acid, maleic acid, fumaric acid, sorbic acid, phthalic acid, terephthalic acid, and derivatives thereof; tricarboxylic acids such as hemimellitic acid, trimellitic acid, trimesic acid, agaric acid, citric acid, 1,2,3-propanetricarboxylic acid, and derivatives thereof; or multicarboxylic acids such as pyromellitic acid, mellitic acid, and derivatives thereof; and polycarboxylic acids such as poly (meth)acrylic acid, and derivatives thereof; and mixtures thereof.

Among these, the polycarboxylic acid is preferable from the viewpoint of having high polymerizability and improving fillability or adhesiveness of the dental composition. It is also preferable to contain the tartaric acid as the acidic component from the viewpoint of moderating the oxidation reaction of the lanthanoid fluoride powder in the dental composition.

Examples of the polycarboxylic acid include, but are not particularly limited to, homopolymers and copolymers of α-β-unsaturated monocarboxylic acid or α-β-unsaturated dicarboxylic acid.

Examples of the α-β-unsaturated monocarboxylic acid or the α-β-unsaturated dicarboxylic acid constituting the homopolymers and copolymers include acrylic acid, methacrylic acid, 2-chloroacrylic acid, 3-chloroacrylic acid, aconitic acid, mesaconic acid, maleic acid, itaconic acid, fumaric acid, glutaconic acid, and citraconic acid.

The polycarboxylic acid may be used alone or in combination of two or more. Among the polycarboxylic acid, polyacrylic acid and polymethacrylic acid are preferable, more preferably polyacrylic acid, from the viewpoint of further improving fillability or adhesiveness of the dental composition.

The weight average molecular weight of the polycarboxylic acid is not particularly limited, but is preferably 5,000 or more and 40,000 or less. The polycarboxylic acid is typically blended in a liquid component, but may be blended in a powder component.

The content of the acidic component is not particularly limited. The content of the acidic component is, for example, 1 mass% or more and 50 mass% or less, preferably 7 mass% or more and 40 mass% or less, and more preferably 10 mass% or more and 35 mass% or less, in the dental composition. When the content of the acidic component in the dental composition is 1 mass% or more and 50 mass% or less, the dental composition can be made into a form of a paste, thereby facilitating handling as the dental composition.

The lanthanoid fluoride powder included in the dental composition of the present embodiment is at least one lanthanoid fluoride powder selected from the group consisting of fluoride powders of lanthanoids having an atomic number of 66 or less.

As used herein, the lanthanoid fluoride powder indicates that the form of the lanthanoid fluoride is powder. When the form of the lanthanoid fluoride in the dental composition is powder, the lanthanoid fluoride is readily blended into the dental composition.

The particle size of the lanthanoid fluoride powder is not particularly limited, but is, for example, 500 um or less, preferably 0.01 um or more and 150 um or less, more preferably 0.05 um or more and 100 um or less, further preferably 0.1 um or more and 25 um or less. Here, the particle size means the average particle size as defined by the median diameter (d50).

When the particle size of the lanthanoid fluoride powder is 500 um or less, the decrease in the strength of the cured body of the dental composition is reduced, and when the particle size is 0.01 um or more and 150 um or less, precipitation and liquid separation can be prevented. When the particle size of the lanthanoid fluoride powder is 100 um or less, the fillability and operability of the dental composition can be improved, and when the particle size is 25 um or less, it is possible to avoid the floating of the prosthesis due to the excessive thickness of the coating when the lanthanoid fluoride powder is blended with a cementing agent.

The lanthanoids having an atomic number of 66 or less is the lanthanoids having an atomic number of 57 to 66. The lanthanoids having an atomic number of 57 to 66 are La (lanthanum), cerium (Ce), praseodymium (Pr), neodymium (Nd), promethium (Pr), samarium (Sm), europium (Eu), gadolinium (Gd), terbium (Tb), and dysprosium (Dy).

The fluoride powders of lanthanoids having an atomic number of 66 or less may be used alone or in combination of two or more.

Among them, powders of fluorides of at least one lanthanoid of lanthanum, cerium, praseodymium, and neodymium (lanthanum fluoride, cerium fluoride, praseodymium fluoride, and neodymium fluoride) is preferable, from the viewpoint of having high acid resistance in the dental composition and high X-ray contrast. Further, lanthanum fluoride, cerium fluoride powder, and neodymium fluoride powder are more preferable, from the viewpoint of the product cost of the dental composition.

The content of the lanthanoid fluoride powder is not particularly limited. The content of the lanthanoid fluoride powder is, for example, 1 mass% or more and 90 mass% or less, preferably 5 mass% or more and 75 mass% or less, more preferably 10 mass% or more and 45 mass% or less, in the dental composition.

When the content of the lanthanoid fluoride powder in the dental composition is 1 mass% or more, the effect of the lanthanoid fluoride powder as an X-ray contrast medium is enhanced. When the content of the lanthanoid fluoride powder in the dental composition is 90 mass% or less, the blending amount of other components in the dental composition can be ensured, and the physical properties of the dental composition can be improved.

The mass ratio of the lanthanoid fluoride powder to the acidic component is not particularly limited, but is, for example, 1 mass% or more and 400 mass% or less, preferably 1 mass% or more and 300 mass% or less, and more preferably 10 to 200 mass%.

When the mass ratio of the lanthanoid fluoride powder to the acidic component is 1 mass% or more, the effect of the lanthanoid fluoride powder as the X-ray contrast medium (hereinafter referred to as X-ray contrast or contrast) is enhanced. When the mass ratio of the lanthanoid fluoride powder to the acidic component is 400 mass% or less, the blending amount of other components including the acidic component can be ensured, and the physical properties of the dental composition can be improved.

When barium glass and lanthanoid fluoride having an atomic number of more than 66 (for example, ytterbium fluoride), which provide X-ray contrast, are used in a dental composition containing the acidic component, they react with the acidic component and cure after a certain period of storage, rendering the dental composition unusable. Even when the glass containing lanthanum, cerium, praseodymium or neodymium is used under the same condition, the glass portion reacts with the acidic component, rendering the dental composition unusable.

In contrast, the dental composition of the present embodiment uses the acidic component and at least one lanthanoid fluoride powder selected from the group consisting of the fluoride powders of the lanthanoids having an atomic number of 66 or less, thereby improving preservability under an acidic environment. Further, in the dental composition of the present embodiment, by improving the preservability under the acidic environment, the function of the lanthanoid fluoride powder as the X-ray contrast medium can be maintained and high X-ray contrast can be maintained.

The dental composition of the present embodiment may further contain a filler other than the lanthanoid fluoride powder. The filler is preferably, but not particularly limited to, aluminosilicate glass, and more preferably fluoroaluminosilicate glass. Here, the fluoroaluminosilicate glass is aluminum and silicon acid fluoride glass, and is glass capable of imparting fluorine sustained-release property.

The composition of the fluoroaluminosilicate glass is not particularly limited, but is, for example, by mass ratio of ions, 10 mass% or more and 33 mass% or less of silicon, 4 mass% or more and 30 mass% or less of aluminum, 5 mass% or more and 36 mass% or less of alkaline earth metals, 0 mass% or more and 10 mass% or less of alkaline metals, 0.2 mass% or more and 16 mass% or less of phosphorus, 2 mass% or more and 40 mass% or less of fluorine, and the remaining amount is oxygen.

As the alkaline earth metal in the fluoroaluminosilicate glass, calcium, magnesium, strontium, and barium are preferable. As the alkaline metal, sodium, lithium, and potassium are preferable, and sodium is more preferable among them. Further, when necessary, a part of the aluminum may be replaced with titanium, yttrium, zirconium, hafnium, tantalum, lanthanum, or the like.

The fluoroaluminosilicate glass may be produced, for example, by a method of mixing a metal compound such as silica, alumina, fluorite, cryolite, aluminum fluoride, and the like, sintering at 1,100 to 1,300°C, and then pulverizing, or by a sol-gel method. The inorganic filler may be surface treated by a known method using a surface treatment agent such as a silane coupling agent.

As the silane coupling agent, methyltrimethoxysilane, methyltriethoxysilane, methyltrichlorosilane, dimethyldichlorosilane, trimethylchlorosilane, vinyltrichlorosilane, vinylethoxysilane, γ-methacryloyloxypropyltrimethoxysilane, γ-chloropropyltrimethoxysilane, hexamethyldisilazane, and the like, are suitably used.

The fluoroaluminosilicate glass may be coated with a polymer compound or the like for the purpose of improving the kneading property with other components.

The content of the aluminosilicate glass is not particularly limited. The content of the fluoroaluminosilicate glass is, for example, 1 mass% or more and 40 mass% or less, preferably 5 mass% or more and 35 mass% or less, more preferably 10 mass% or more and 30 mass% or less, in the dental composition.

When the content of the fluoroaluminosilicate glass in the dental composition is 1 mass% or more, fluorine sustained-release property can be imparted to the dental composition. When the content of the fluoroaluminosilicate glass in the dental composition is 40 mass% or less, the blending amount of other components in the dental composition can be ensured, and the physical properties of the dental composition can be improved.

The dental composition of the present embodiment may contain other components as long as the object of the present invention is not impaired. Examples of the other components contained in the dental composition include a polymerizable monomer, a polymerization initiator, a polymerization inhibitor, a viscosity modifier, and a solvent.

The polymerizable monomer is preferably, but not particularly limited to, a radical polymerizable monomer, for example, and more preferably (meth)acrylate.

As used herein, the (meth)acrylate means acrylate and/or methacrylate. The (meth)acrylate refers to an alkyl ester of (meth)acrylic acid having one or more (meth)acryloyloxy group. Here, the (meth)acryloyloxy group means a methacryloyloxy group and/or an acryloyloxy group.

Examples of the radical polymerizable monomer include α-cyanoacrylate esters, (meth)acrylate esters, α-haloacrylate esters, crotonate esters, cinnamate esters, sorbate esters, maleate esters, itaconate esters, (meth)acrylamides, (meth)acrylamide derivatives, vinyl ester, vinyl ether, mono-N-vinyl derivatives, styrene derivatives, and the like.

The radical polymerizable monomer may be used alone or in combination of two or more. Among these, the (meth)acrylate esters and the (meth)acrylamide derivatives are preferable, and the (meth)acrylate esters are more preferable.

Examples of a monofunctional radical polymerizable monomer include methyl (meth)acrylate, isobutyl (meth)acrylate, benzyl (meth)acrylate, lauryl (meth)acrylate, 2,3-dibromopropyl (meth)acrylate, 2-hydroxyethyl (meth)acrylate, 6-hydroxyhexyl (meth)acrylate, 10-hydroxydecyl (meth)acrylate, propylene glycol mono(meth)acrylate, glyceryl mono(meth)acrylate, erythritol mono(meth)acrylate, N-methylol (meth)acrylamide, N-hydroxyethyl (meth)acrylamide, N-dihydroxyethyl (meth)acrylamide, (meth)acryloyloxydodecylpyridinium bromide, (meth)acryloyloxydodecylpyridinium chloride, (meth)acryloyloxyhexadecylpyridinium chloride, (meth)acryloyloxyhexadecylpyridinium chloride, (meth)acryloyloxidecylammonium chloride, and the like.

Examples of a bifunctional radical polymerizable monomer include ethylene glycol di(meth)acrylate, triethylene glycol di(meth)acrylate, propylene glycol di(meth)acrylate, neopentyl glycol di(meth)acrylate, 1,6-hexanediol di(meth)acrylate, 1,10-decanediol di(meth)acrylate, 2,2-bis[4-[3-(meth)acryloyloxy-2-hydroxypropoxy] phenyl] propane, 2,2-bis[4-(2-(meth)acryloyloxy ethoxy) phenyl] propane, 2,2-bis[4-(meth)acryloyloxy polyethoxyphenyl] propane, 1,2-bis[3-(meth)acryloyloxy-2-hydroxypropoxy] ethane, pentaerythritol di(meth)acrylate, [2,2,4-trimethylhexamethylenebis (2-carbamoyloxyethyl)] di(meth)acrylate, and the like.

Examples of a trifunctional or more radical polymerizable monomer include trimethylolpropane tri(meth)acrylate, trimethylolethane tri(meth)acrylate, tetramethylolmethane tri(meth)acrylate, pentaerythritol tetra(meth)acrylate, dipentaerythritol hexa(meth)acrylate, N,N'-(2,2,4-trimethylhexamethylene) bis[2-(aminocarboxy) propane -1,3-diol] tetramethacrylate, 1,7-diacryloyloxy-2,2,6,6-tetraacryloyloxymethyl-4 oxyheptane, and the like.

The polymerizable monomer may be used alone or in combination of two or more.

The content of the polymerizable monomer in the dental composition is not particularly limited and may be, for example, 0.1 mass% or more and 50 mass% or less, preferably 0.5 mass% or more and 35 mass% or less, and more preferably 1 mass% or more and 25 mass% or less, in the dental composition. When the content of the polymerizable monomer in the dental composition is 0.1 mass% or more, the adhesiveness of the dental composition to the tooth is further improved, and when the content is 50 mass% or less, the blending amount of other components in the dental composition can be ensured, and the physical properties of the dental composition can be improved.

Examples of the polymerization initiator include a chemical polymerization initiator and a photopolymerization initiator.

Examples of the chemical polymerization initiator include, but are not particularly limited to, thiourea derivatives, vanadium compounds, tertiary amines, and organic peroxides.

The thiourea derivatives, among the chemical polymerization initiator, function as a reducing agent.

Examples of the thiourea derivative include, but are not particularly limited to, ethylenethiourea, N-methylthiourea, N-ethylthiourea, N-propylthiourea, N-butylthiourea, N-laurylthiourea, N-phenylthiourea, N-cyclohexylthiourea, N,N-dimethylthiourea, N,N-diethylthiourea, N,N-dipropylthiourea, N,N-dibutylthiourea, N,N-dilaurylthiourea, N,N-diphenylthiourea, N,N-dicyclohexylthiourea, trimethylthiourea, tetramethylthiourea, N-acetylthiourea, N-benzoylthiourea, 1-allyl-3-(2-hydroxyethyl)-2-thiourea, 1-(2-tetrahydrofurfuryl)-2-thiourea, N-tert-butyl-N'-isopropylthiourea, 2-pyridylthiourea, and the like.

The thiourea derivative may be used alone or in combination of two or more. Among them, N-benzoylthiourea is preferable in terms of improving the curability of the dental composition.

The content of the thiourea derivative in the dental composition is preferably, but is not particularly limited to, 0.1 mass% or more and 10 mass% or less, more preferably 0.1 mass% or more and 5 mass% or less, further preferably 0.1 mass% or more and 2 mass% or less, in the dental composition. When the content of the thiourea derivative in the dental composition is 0.1 mass% or more, the curability of the dental composition is further improved, and when the content is 10 mass% or less, the solubility of the thiourea derivative in (meth)acrylate in the dental composition is improved.

The vanadium compound, among the chemical polymerization initiator, functions as a reducing agent.

Examples of the vanadium compound include, but are not particularly limited to, oxovanadium oxalate, vanadyl acetylacetonate, vanadium acetylacetonate, vanadyl stearate, vanadium naphthenate, vanadium benzoylacetonate, and the like. The vanadium compound may be used alone or in combination of two or more. Among these, vanadyl acetylacetonate is preferable in terms of the curability of the dental composition.

The content of the vanadium compound in the dental composition is not particularly limited, but is preferably 0.001 mass% or more and 10 mass% or less, more preferably 0.0015 mass% or more and 5 mass% or less, and further preferably 0.002 mass% or more and 3 mass% or less. When the content of the vanadium compound in the dental composition is 0.001 mass% or more, the curability of the dental composition is further improved, and when the content is 10 mass% or less, the storage stability of the dental composition is further improved.

The tertiary amine, among the chemical polymerization initiator, functions as a reducing agent.

Examples of the tertiary amine include, but are not particularly limited to, a tertiary aliphatic amine and a tertiary aromatic amine.

Examples of the tertiary aliphatic amine include N,N-dimethylaminoethyl methacrylate, triethanolamine, and the like.

Examples of the tertiary aromatic amine include alkyl p-dialkylaminobenzoate, 7-dimethylamino -4-methylcoumarin, N,N-dimethylaniline, N,N-dibenzylaniline, N,N-dimethyl-p-toluidine, N,N-diethyl-p-toluidine, N,N-bis(2-hydroxyethyl)-p-toluidine, N,N,2,4,6-pentamethylaniline, N,N,2,4-tetramethylaniline, N,N-diethyl-2,4,6-trimethylaniline, and the like.

Among these, the tertiary amine is preferably the tertiary aromatic amine, and more preferably alkyl p-dialkylaminobenzoate.

Examples of the alkyl p-dialkylaminobenzoate include methyl p-dimethylaminobenzoate, ethyl p-dimethylaminobenzoate, propyl p-dimethylaminobenzoate, amyl p-dimethylaminobenzoate, isoamyl p-dimethylaminobenzoate, ethyl p-diethylaminobenzoate, propyl p-diethylaminobenzoate, and the like.

The tertiary amine may be used alone or in combination of two or more.

The organic peroxide, among the chemical polymerization initiator, functions as an oxidant.

Examples of the organic peroxide include benzoyl peroxide, cumene hydroperoxide, t-butyl hydroperoxide, t-amyl hydroperoxide, 1,1,3,3-tetramethylbutyl hydroperoxide, 2,5-dimethyl-2,5-di(hydroperoxy) hexane, p-diisopropylbenzene monohydroperoxide, p-methane hydroperoxide, pinane hydroperoxide, and the like.

The organic peroxide may be used alone or in combination of two or more. Among these, cumene hydroperoxide is preferable in terms of the curability of the dental composition.

The content of the organic peroxide in the dental composition is not particularly limited, but is preferably, for example, 0.01 mass% or more and 10 mass% or less, more preferably 0.05 mass% or more and 5 mass% or less, more preferably 0.1 mass% or more and 3 mass% or less. When the content of the organic peroxide in the dental composition is 0.01 mass% or more, the curability of the dental composition is further improved, and when the content is 10 mass% or less, the working time with the dental composition is increased, thereby ensuring sufficient working time.

Examples of the photopolymerization initiator include, but are not particularly limited to, camphorquinone, phenylbis(2,4,6-trimethylbenzoyl) phosphine oxide, 2,4,6-trimethylbenzoyldiphenylphosphine oxide, benzylketal, diacetylketal, benzyldimethylketal, benzyldiethylketal, benzylbis(2-methoxyethyl) ketal, 4,4'-dimethyl (benzyldimethylketal), anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluoromethylthioxanthone, thioxanthone-10,10-dioxide, thioxanthone-10-oxide, benzoin methyl ether, benzoin ethyl ether, benzoin isopropyl ether, benzoin isobutyl ether, benzophenone, bis(4-dimethylaminophenyl) ketone, 4,4'-bis(diethylamino) benzophenone, and the like.

The photopolymerization initiator may be used alone or in combination of two or more. Among them, camphorquinone and phenylbis(2,4,6-trimethylbenzoyl) phosphine oxide are preferable in terms of improving the curability of the dental composition.

The content of the photopolymerization initiator in the dental composition is not particularly limited, but is preferably 0.001 mass% or more and 10 mass% or less, more preferably 0.005 mass% or more and 5 mass% or less, and further preferably 0.01 mass% or more and 33 mass% or less. When the content of the photopolymerization initiator in the dental composition is 0.001 mass% or more, the curability of the dental composition is further improved, and when the content is 10 mass% or less, the storage stability of the dental composition is further improved.

Examples of the polymerization inhibitor include dibutylhydroxytoluene (2,6-di-tert-butyl-p-cresol), 6-tert-butyl-2,4-xylenol, and the like.

The polymerization inhibitor may be used alone or in combination of two or more. Among them, dibutylhydroxytoluene is preferable in terms of improving the curability of the dental composition.

The content of the polymerization inhibitor in the dental composition is not particularly limited, but when the polymerization inhibitor is contained, the content may be 0.001 mass% or more and 5 mass% or less, preferably 0.005 mass% or more and 1 mass% or less, more preferably 0.01 mass% or more and 0.1 mass% or less. When the content of the polymerization inhibitor in the dental composition is 0.001 mass% or more and 5 mass% or less, the storage stability of the dental composition is improved.

The viscosity modifier is not particularly limited, but is, for example, a fine filler having a particle size of 0.07 um or less, and preferably 0.5 um or more and 0.05 um or less. Examples of the fine filler include silica fine powder, alumina fine powder, and sodium salt of carboxymethyl cellulose (CMC-Na). The viscosity modifier may be used alone or in combination of two or more.

The content of the viscosity modifier in the dental composition is not particularly limited, but for example, 0.01 mass% or more and 25 mass% or less, preferably 0.1 mass% or more and 20 mass% or less, more preferably 1 mass% or more and 15 mass% or less, in the dental composition. When the blending amount of the viscosity modifier in the dental composition is 0.01 mass% or more and 25 mass% or less, the mechanical strength of the cured product of the dental composition is improved, and the lowering of the surface smoothness is reduced.

Examples of the solvent include, but are not particularly limited to, water and an organic solvent.

Examples of the organic solvent include ethanol, acetone, propanol, glycerin, and the like.

The solvent may be used alone or in combination of two or more. Among them, a solvent in which glycerin is mixed with water is preferable, from the viewpoint of not reacting with the oxide powder and improving the wettability of the dental composition to the tooth.

The content of the solvent is not particularly limited, but is, for example, 5 mass% or more and 50 mass% or less, preferably 10 mass% or more and 40 mass% or less, more preferably 20 mass% or more and 35 mass% or less, in the dental composition.

The dental composition of the present embodiment may be constituted as a dental composition containing a first agent and a second agent.

The first agent contains an acidic component and an agent containing the lanthanoid fluoride powder described above.

The acidic component in the first agent is not particularly limited, but is, for example, the acidic component described above. Specifically, the acidic component in the first agent is the polycarboxylic acid described above, and preferably the polyacrylic acid described above.

The content of the acidic component in the first agent is not particularly limited, but is, for example, 1 mass% or more and 50 mass% or less, preferably 7 mass% or more and 40 mass% or less, and more preferably 10 mass% or more and 35 mass% or less, in the first agent. When the content of the acidic component in the first agent is 1 mass% or more and 50 mass% or less, the blending amount of the other components can be ensured, and the physical properties as the dental composition can be improved.

The lanthanoid fluoride powder in the first agent is not particularly limited, but the lanthanoid fluoride powder described above is used. Specifically, the lanthanoid fluoride powder in the first agent is at least one lanthanoid fluoride powder selected from the group consisting of the fluoride powders of lanthanoids having an atomic number of 66 or less, and is preferably the fluoride powders of lanthanoids of at least one of lanthanum, cerium, praseodymium, and neodymium, as described above.

The content of the lanthanoid fluoride powder in the first agent is not particularly limited. The content of the lanthanoid fluoride powder in the first agent is, for example, 1 mass% or more and 90 mass% or less, preferably 5 mass% or more and 75 mass% or less, more preferably 10 mass% or more and 45 mass% or less.

When the content of the lanthanoid fluoride powder in the first agent is 1 mass% or more, the effect of the lanthanoid fluoride powder as an X-ray contrast medium (X-ray contrast) is enhanced. When the content of the lanthanoid fluoride powder in the first agent is 90 mass% or less, the blending amount of other components in the first agent can be ensured, and the physical properties as a dental composition can be improved.

The first agent may contain, as other components, the polymerization initiator, the viscosity modifier, and the solvent.

As the first agent, for example, an agent constituting the dental composition described above may be used.

The first agent may be made into a form of a paste by containing a polycarboxylic acid. Therefore, handling of the first agent can be facilitated as an agent constituting the dental composition.

The second agent contains aluminosilicate glass and a (meth)acrylate.

The aluminosilicate glass in the second agent is not particularly limited, but for example, the aluminosilicate glass described above is used. Specifically, the aluminosilicate glass in the second agent is fluoroaluminosilicate glass.

The content of the aluminosilicate glass in the second agent is not particularly limited. The content of the fluoroaluminosilicate glass is, for example, 10 mass% or more and 85 mass% or less, preferably 15 mass% or more and 80 mass% or less, more preferably 20 mass% or more and 75 mass% or less, in the second agent.

When the content of the fluoroaluminosilicate glass in the second agent is 10 mass% or more, sufficient fluorine sustained-release property can be imparted to the dental composition containing the second agent. When the content of the fluoroaluminosilicate glass in the second agent is 85 mass% or less, the blending amount of other components in the dental composition can be ensured, and the physical properties of the dental composition can be improved.

The (meth)acrylate in the second agent is not particularly limited, but for example, the polymerizable monomer included in the other components described above is used. Specifically, the (meth)acrylate in the second agent is the polymerizable monomer being the other component described above, preferably the (meth)acrylate described above.

The content of the (meth)acrylate in the second agent is not particularly limited but may be, for example, 0.1 mass% or more and 50 mass% or less, preferably 0.5 mass% or more and 40 mass% or less, more preferably 1 mass% or more and 30 mass% or less, in the second agent. When the content of the polymerizable monomer in the second agent is 0.1 mass% or more, the adhesiveness of the dental composition containing the second agent to the tooth is further improved, and when the content is 50 mass% or less, the blending amount of other components in the dental composition can be ensured, and the physical properties of the dental composition can be improved.

The second agent may contain, as other components, the polymerization inhibitor, the viscosity modifier, and the solvent.

The second agent may be made into a form of a paste by containing the (meth)acrylate. Therefore, handling of the second agent can be facilitated as another agent constituting the dental composition.

The mass ratio of the first agent to the second agent of the dental composition of the present embodiment is, for example, 10:1 to 1:10, preferably 1:5 to 5:1, more preferably 1:3 to 3:1. When the mass ratio of the first agent to the second agent is 10:1 to 1:10, the ratio of the reactive component in the composition is appropriate and contributes to the improvement of physical properties.

In the dental composition of the present embodiment, the first agent and the second agent may be kneaded and used.

In the present embodiment, in a case where the dental composition includes the first agent and the second agent as described above, the first agent uses the acidic component and at least one lanthanoid fluoride powder selected from the group consisting of the fluoride powders of the lanthanoids having an atomic number of 66 or less, thereby improving preservability under an acidic environment and ensuring that high X-ray contrast can be maintained.

The application of the dental composition of the present embodiment is not particularly limited, but it can be used for various dental materials, for example. Examples of the dental materials include dental cement, dental adhesive, dental temporary sealing material, dental primer, dental coat material, dental composite resin, dental hard resin, dental cutting resin material, dental temporary restorative material, dental filler, dentifrice, and the like. Among these, the dental composition of the present embodiment is suitably used for the dental cement.

### Example

Hereinafter, the present invention will be further described with reference to Examples. In the following, numerical values or "%" without units are based on mass (parts by mass or % by mass) unless otherwise specified.

### <Preparation of First Agent>

### [Examples 1 to 6 and Comparative Examples 1 and 2]

A paste of a first agent (hereinafter referred to as a first paste) was obtained by mixing an X-ray contrast medium, an acidic component, a polymerization initiator, a viscosity modifier, and a solvent (water), with the composition (parts by mass) presented in Table 1.

### <Preparation of Second Preparation>

### [Blending Example 1 and 2]

A paste 2 of a second agent (hereinafter referred to as a second paste) was obtained by mixing fluoroaluminosilicate glass, a polymerizable monomer, a polymerization inhibitor, a viscosity modifier, and a solvent (water), with the composition (parts by mass) presented in Table 2.

| | | EXAMPLE 1 | EXAMPLE 2 | EXAMPLE 3 | EXAMPLE 4 | EXAMPLE 5 | EXAMPLE 6 | COMPARATIVE EXAMPLE 1 | COMPARATIVE EXAMPLE 2 |
|---|---|---|---|---|---|---|---|---|---|
| X-RAY CONTRAST MEDIUM | LANTHANUM FLUORIDE (MEDIAN 0.3 *µ*m) | 20 | | 41 | | | | | |
| | LANTHANUM FLUORIDE (MEDIAN 9 *µ*m) | | 20 | | | | 31 | | |
| | YTTERBIUM FLUORIDE | | | | | | | 20 | |
| | CERIUM FLUORIDE | | | | 20 | | | | |
| | NEODYMIUM FLUORIDE | | | | | 20 | | | |
| | BARIUM-CONTAINING GLASS | | | | | | | | 20 |
| ACIDIC COMPONENT | POLYACRYLIC ACID | 32 | 32 | 24 | 32 | 32 | 15 | 32 | 32 |
| | TARTARIC ACID | 8 | 8 | 7 | 8 | 8 | 1 | 8 | 8 |
| POLYMERIZATION INITIATOR | L-CYSTE I NE HYDROCHLORIDE | 2 | 2 | 1 | 2 | 2 | | 2 | 2 |
| VISCOSITY MODIFIER | SILICA FINE POWDER | 6 | 6 | 3 | 6 | 6 | 4 | 6 | 6 |
| FILLER | SILICON DIOXIDE | | | | | | 17 | | |
| SOLVENT | GLYCERIN | | | | | | 10 | | |
| | ION EXCHANGED WATER | 32 | 32 | 24 | 32 | 32 | 22 | 32 | 32 |
| TOTAL | | 100 | 100 | 100 | 100 | 100 | 100 | 100 | 100 |
| EVALUATION | PRESERVABILITY | A | A | A | A | A | A | B | B |
| | X-RAY CONTRAST | A | A | A | A | A | A | A | A |

**[Table 2]**

| COMPONENT | COMPOUND NAME | BLENDING EXAMPLE 1 | BLENDING EXAMPLE 2 |
|---|---|---|---|
| FLUOROALUMINOSILICATE GLASS | FLUOROALUMINOSILICATE GLASS I SILANE-TREATED POWDER | 70 | 56 |
| POLYMERIZABLE MONOMER | HYDROXYETHYL METHACRYLATE | 5 | |
| | BISPHENOL A ETHOXY-MODIFIED DIMETHACRYLATE (n=30) | 3 | |
| | DI-2-METHACRYL0XYETHYL-2, 2, 4-TRIETHYLHEXAMETHYLENE DICARBAMATE | 5 | |
| | GLYCERIN DIMETHACRYLATE | 5 | |
| | BISPHENOL A ETHOXY-MODIFIED DIMETHACRYLATE (n=10) | 11.97 | |
| POLYMERIZATION INHIBITOR | BUTYLATED HYDROXYTOLUENE | 0.03 | |
| VISCOSITY MODIFIER | SILICA FINE POWDER | | 2 |
| | ALUMINA FINE POWDER | | 1 |
| | CMC-Na | | 1 |
| SOLVENT | ION EXCHANGED WATER | | 20 |
| | GLYCERIN | | 20 |
| TOTAL | | 100 | 100 |

The storage stability of the first paste was checked as follows.

### <Storage Stability>

Approximately 2 g of the first paste is taken onto a piece of mixing paper and the paste is kneaded and loosened using a plastic spatula over a wide area of the mixing paper for approximately 15 seconds. The loosened paste is measured using a glass tube for consistency measurement (10 mm inner diameter, marked at the position of 0.5 ml) and gently pressed out onto a plastic sheet.

Over them, a plastic sheet, a glass plate (approximately 20 g in weight), and a weight (approximately 100 g in weight) are placed so that the total weight is 120±0.5 g. After 2 minutes from the time of loading, the weight and the glass plate are removed and the average of the long and short diameters of the spread sample is taken as the initial consistency.

The prepared first paste is evaluated according to the following criteria. When the evaluation is A, the preservability is determined to be good, and when the evaluation is B, the preservability is determined to be poor. The results of the storage stability are presented in Table 1.

### [Evaluation Criteria]

A: The consistency immediately after preparation is 25 mm or more, and the re-measured value of the consistency after 2 weeks of standing at 23°C from preparation is 80% or more of the initial value.
B: The consistency immediately after preparation is less than 25 mm, or the re-measured value of the consistency after 2 weeks of standing at 23°C from preparation is less than 80% of the initial value.

The first paste and the second paste were mixed and kneaded at a mass ratio of 1:1 to obtain a cured body. The X-ray contrast of the cured body was checked as follows. The X-ray contrast of Example 1 to 5 and Comparative Examples 1 and 2 is the X-ray contrast when the first paste of Example 1 to 5 are mixed with the second paste of Blending Example 1. The X-ray contrast of Example 6 is the X-ray contrast when the first paste of Example 6 is mixed with the second paste of Blending Example 2.

### <X-ray Contrast>

A digital X-ray unit or a single-phase dental X-ray unit is used, as a diagnostic integrated X-ray generator specified in JIS Z 4711, or as a device capable of operating at a tube voltage of 65±5 kV and with the ability to fully penetrate a 1.5 mm thick aluminum plate.

These are sized for the film of the X-ray sensor occlusal method and are calibrated for using the digital X-ray unit. For example, they are calibrated for use with, for example, a charge-coupled device (CCD), a luminescent fluorescent plate (image plate), and a single-phase dental X-ray unit with appropriate software. Further, as software capable of gradation analysis, software suitable for gradation analysis is used.

An aluminum step wedge made of aluminum with a purity of 98% or more mass fraction (mass fraction of copper is less than 0.1% and mass fraction of iron is less than 1.0%), having an overall dimension of 50 mm in length and 20 mm in width, and having a thickness range of 0.5 to 5.0 mm with equally spaced steps of 0.50±0.01 mm, is used. The wedge is placed such that the stepped surfaces of all thicknesses are parallel to the X-ray film and are perpendicular to X-rays.

A mold capable of producing a specimen of 15±1 mm in diameter and 1.0±0.1 mm or 1.00±0.01 mm in thickness, is used. A film that is transparent and is 50±30 um in thickness (for example, a film made of polyester that does not adhere to the sample and is not rough), is used. A glass slide or plate made of glass, stainless steel, or other smooth and hard material, is used.

A thermo-hygrostat capable of maintaining a temperature of 37±1°C and a humidity of 50% or more, is used. A micrometer or equivalent, specified in JIS B 7502, with a reading of 0.01 mm or more, is used. A clip or equivalent (for example, one that can hold the mold while cement hardens) is used as a clamp.

An abrasive paper conforming to JIS R 6252 or JIS R 6253 (P2000 or P2500) is used. A lead sheet having a thickness of 2.0 mm or more is used. Furthermore, a light irradiator is used.

A film is placed on the glass slide or plate and the mold is placed on it. A cement kneaded material is extruded and slightly overfilled into the mold. Another film is placed on the cement, and another glass slide or plate is placed over it to push out excess cement. This is pressurized with the clamp. After 3 minutes from the start of kneading, the whole product is placed in the thermo-hygrostat maintained at 37±1°C for 30 minutes.

The specimen is removed from the mold and the thickness near the center of the disk is measured using the micrometer. Only specimens with a thickness of 1.0±0.1 mm are measured. When the specimen is too thick, it may be polished to the specified thickness using the abrasive paper. The specimen is placed in distilled or purified water at 23±1°C and tested within 7 days. To prevent the specimen from drying, the specimen is tested within 30 minutes of removal from the water.

As a measurement procedure, before imaging, the automatic density correction function is disabled by using the software of the digital X-ray equipment. The thickness of the specimen (TS) is measured with an accuracy of 0.01 m using the micrometer. The X-ray sensor is placed on the lead sheet. The specimen and the step wedge are placed in the center of the sensor. Without using the automatic density correction function, the distance between the cathode and the X-ray sensor is set to 400 mm, and the specimen and the step wedge are irradiated with X-rays.

The irradiation is repeated with different irradiation times, and a clear image is obtained. The digital image file is transferred to the software that can analyze gray values. Using a software measurement tool that can analyze gray values, a rectangular area is defined in the image of the specimen, and the average gray value within the area is measured. This procedure is then performed for each step image of the step wedge.

The gray value of each step wedge is plotted against the thickness of each step, and the relationship between the aluminum thickness and the gray value is determined. The aluminum thickness (Ta) relative to the gray value of the specimen with thickness (TS), is determined. The X-ray contrast (equivalent to aluminum) value of a unit thickness (1.0 mm) of the specimen is determined by dividing by Ta and TS. The results of the X-ray contrast are presented in Table 1.

From Table 1, the dental composition (the first agent and the second agent) including an agent containing an acidic component and at least one lanthanoid fluoride powder selected from the group consisting of fluoride powders of lanthanoids having an atomic number of 66 or less (Examples 1 to 5 and Example 6), exhibited good storage stability and X-ray contrast.

In contrast, when at least one lanthanoid fluoride powder selected from the group consisting of fluoride powders of lanthanoids having an atomic number of 66 or less was not included, and when ytterbium fluoride was contained instead of at least one lanthanoid fluoride powder selected from the group consisting of fluoride powders of lanthanoids having an atomic number of 66 or less, storage stability was poor (Comparative Examples 1 and 2).

While embodiments of the present invention have been described, the present invention is not limited to specific embodiments, and various modifications and variations are possible within the scope of the invention as claimed.

The present application is based on and claims priority to Japanese Patent Application No. 2021-162412, filed September 30, 2021, the contents of which are incorporated herein by reference in their entirety.

## Claims

1. A dental composition comprising:
an acidic component; and
at least one lanthanoid fluoride powder selected from the group consisting of fluoride powders of lanthanoids having an atomic number of 66 or less.

2. The dental composition according to claim 1, wherein the lanthanoid fluoride powder is lanthanum fluoride powder, cerium fluoride powder, praseodymium fluoride powder, or neodymium fluoride powder.

3. The dental composition according to claim 1 or 2, wherein the acidic component is a polycarboxylic acid.

4. The dental composition according to any one of claims 1 to 3, wherein the dental composition is in a form of a paste.

5. The dental composition according to any one of claims 1 to 4, further comprising aluminosilicate glass.

6. A dental composition including a first agent and a second agent, wherein
the first agent contains:
an acidic component; and
at least one lanthanoid fluoride powder selected from the group consisting of fluoride powders of lanthanoids having an atomic number of 66 or less.

7. The dental composition according to claim 6, wherein
the lanthanoid fluoride powder is lanthanum fluoride powder, cerium fluoride powder, praseodymium fluoride powder, or neodymium fluoride powder,
the first agent further contains a polyacrylic acid, and
the second agent contains aluminosilicate glass and a (meth)acrylate.
